# FASCICULE DE BREVET EUROPEEN

(11) **EP 3 007 822 B1**
(45) Date de publication et mention de la délivrance du brevet: **22.01.2020**
(21) Numéro de dépôt: 14731800.0
(22) Date de dépôt: 26.05.2014
(51) Int. Cl.: B01J 23/50, B01J 37/03, B01J 37/16, C07C 51/16, C07C 59/06, C07C 59/01, C07C 51/235, C07C 51/23, C07C 59/10

(54) **PROCÉDÉ DE SYNTHÈSE D'ACIDE GLYCOLIQUE**
HERSTELLUNGSVERFAHREN VON GLYCOLSÄURE
PROCESS FOR PREPARING GLYCOLIC ACID

(30) Priorité: 10.06.2013 FR 1355321
(43) Date de publication de la demande: 20.04.2016
(73) Titulaire: Pivert, 60280 Venette (FR); Centre National de la Recherche Scientifique (C.N.R.S.), 75794 Paris Cedex 16 (FR); Université de Lille, 59800 Lille (FR)
(72) Inventeur: SKRZYNSKA, Elzbieta, 30-683 Cracow (PL); DUMEIGNIL, Franck, 59491 VILLENEUVE D'ASCQ (FR); CAPRON, Mickaël, 59830 Bachy (FR); DUHAMEL, Louise, 59650 Villeneuve d'Ascq (FR)
(74) Mandataire: McDade, Sophie V.G.A.
(86) Numéro de dépôt international: PCT/IB2014/061725
(87) Numéro de publication internationale: WO 2014/199256

(56) Documents cités:
- EP-A1- 0 091 165
- CN-A- 1 775 351
- CN-A- 101 279 911
- CN-A- 101 284 774
- FR-A1- 2 424 264
- JP-A- S59 112 838
- US-A- 4 548 921
- US-A- 5 274 187
- KETCHIE ET AL: "Influence of gold particle size on the aqueous-phase oxidation of carbon monoxide and glycerol", JOURNAL OF CATALYSIS, ACADEMIC PRESS, DULUTH, MN, US, vol. 250, no. 1, 26 juillet 2007 (2007-07-26), pages 94-101, XP022169826, ISSN: 0021-9517, DOI: 10.1016/J.JCAT.2007.06.001
- Satoshi Sato ET AL: "Vapor-phase Dehydration of Glycerol into Hydroxyacetone over Silver Catalyst", Chemistry Letters, vol. 41, no. 9, 5 September 2012 (2012-09-05), pages 965-966, XP055453218, JAPAN ISSN: 0366-7022, DOI: 10.1246/cl.2012.965

## Description

### Domaine de l'invention

La présente invention porte sur un procédé permettant l'oxydation du glycérol en acide glycolique par voie catalytique. La présente invention concerne également un catalyseur à base d'argent et l'utilisation d'un catalyseur à base d'argent pour la synthèse d'acide glycolique à partir du glycérol.

### Contexte de l'invention

L'acide glycolique de formule CH₂(OH)COOH, également appelé acide hydroxyacétique, est utilisé comme monomère dans la préparation de l'acide polyglycolique ainsi que dans celles d'autres copolymères biocompatibles tels que des copolymères polylactides-co-glycolides (PLGA). Parmi les autres utilisations de l'acide glycolique, on peut mentionner son emploi dans l'industrie textile comme agent séchant et colorant. L'acide glycolique peut également entrer dans la composition de certains plats cuisinés en tant que conservateur ou exhausteur de goût. L'acide glycolique est souvent introduit dans les émulsions polymères, les solvants et dans les additifs pour les encres afin d'en améliorer la brillance et la fluidité. Il est également utilisé en cosmétique, grâce à son excellente capacité à pénétrer la peau, le plus souvent dans les peelings. Il peut réduire les rides, l'acné ou l'hyperpigmentation.

Au niveau industriel l'acide glycolique est synthétisé à partir de composés d'origine fossile et est généralement préparé par réaction entre l'acide chloroacétique et l'hydroxyde de sodium suivant la réaction suivante : ClCH₂CO₂H + NaOH → HOCH₂CO₂H + NaCl.

D'autres voies alternatives non industrialisées à nos jours sont connues, comme la réaction de carbonylation entre le formaldéhyde et le gaz de synthèse (CO+H₂). Il peut également être envisagé d'utiliser une synthèse via l'hydrogénation de l'acide oxalique. L'acide glycolique peut aussi être préparé par voie enzymatique.

Les études présentées dans la littérature montrent qu'une forte sélectivité en acide glycolique n'est pas observée pour les catalyseurs couramment utilisés, tels que des catalyseurs à base d'or, de platine ou de palladium. Généralement, le produit majoritaire issu de la réaction, catalysée ou non, d'oxydation du glycérol est l'acide glycérique (CH₂(OH)CH(OH)COOH).

Ketchi et al. « Influence of gold particle size on aqueous-phase oxydation of carbon monoxide and glycérol » décrit l'utilisation de poudre d'argent comme catalyseur de l'oxydation du glycol. La concentration [OH]/[gly] utilisée est de 2. L'argent est considéré comme significativement moins actif que l'or pour la production d'acide glycérique par oxydation du glycérol. L'acide glycolique est le produit secondaire de la réaction étudiée.

De plus les synthèses d'acide glycolique présentées dans la littérature sont réalisées en présence de bases, (par exemple NaOH). La base est généralement utilisée en large excès avec un rapport [base]/[glycérol] de l'ordre de 4. Cette quantité de base ajoutée induit la production de quantités importantes de sels lors de l'acidification nécessaire pour la récupération des molécules cibles. Ces quantités augmentent non seulement les coûts de production mais également l'impact environnemental de la synthèse.

### Description de l'invention

La présente invention a donc pour but de remédier à un ou plusieurs de ces inconvénients, notamment en fournissant une alternative à l'utilisation de ressources d'origine fossile pour la production d'acide glycolique, par l'utilisation de glycérol biosourcé.

Un autre but de l'invention est d'obtenir la synthèse d'acide glycolique à partir du glycérol avec une bonne sélectivité et/ou une bonne valeur de conversion.

Enfin, l'invention porte également sur un nouveau type de catalyseur à base d'argent sur support pour la synthèse de composés organiques.

Selon un premier mode de réalisation, l'invention porte sur l'utilisation d'un catalyseur à base d'argent sur support dans la synthèse d'acide glycolique à partir de glycérol. Ainsi l'invention a notamment pour objet un procédé de synthèse d'acide glycolique, ou d'un de ses sels, comprenant l'étape suivante :
-une mise en contact dans un milieu réactionnel de glycérol et d'un agent oxydant en présence d'un catalyseur à base d'argent sur support
ledit support comprenant un matériau choisi dans le groupe constitué par le CeO₂, l'Al₂O₃ basique éventuellement dopée avec un oxyde de calcium ou de cérium, une résine amphotère, le ZrO₂, et un mélange de ces matériaux ; et
dans lequel une base produisant des ions hydroxyles est présente dans le milieu réactionnel, et le rapport molaire d'ions hydroxyles et de glycérol [OH]/[gly] mis en présence étant de 0,5 à 1,2 ;
et ledit catalyseur ne comprenant essentiellement que de l'argent comme espèce métallique.

Par catalyseur à base d'argent sur support il est désigné des composés comprenant de l'argent sous forme métallique déposé sur un (ou plusieurs) autre(s) matériau(x) servant de support. De préférence, le catalyseur ne comprend que de l'argent comme espèce métallique ou est le composant principal du catalyseur. De préférence le catalyseur sur support ne comprend pas d'autres métaux nobles (en particulier le platine et l'or, et possiblement le rhodium, l'osmium, le palladium, le ruthénium et l'iridium) ou en comprend une faible quantité. Par « faible quantité » on entend pas plus de 5% de la quantité totale de métal (par exemple Ag), de préférence pas plus de 2,5% et encore plus préférentiellement pas plus de 1%.

Il est avantageux que soit présent dans le milieu réactionnel une base produisant des ions hydroxyles, telle que de la soude, de l'hydroxyde de potassium, de l'ammoniac ou leurs mélanges. L'introduction d'une base permet notamment de maintenir un pH élevé. Le pH du milieu réactionnel est préférablement supérieur ou égal à 8, avantageusement supérieur ou égal à 9, par exemple supérieur, ou égal, à 10.

Il est décrit dans le présent texte, l'utilisation d'un rapport molaire d'ions hydroxyles et de glycérol [OH]/[gly] mis en présence pouvant être d'environ 4 ou d'au moins 4.

Le procédé selon l'invention peut avantageusement être effectué en présence d'une concentration de base, dont le rapport [OH]/[gly] est inférieur ou égal à 1. Le rapport [OH]/[gly] est néanmoins avantageusement supérieur à 0,4 de manière à permettre des taux de conversion suffisants, par exemple supérieurs à 5. Ce rapport est avantageusement choisi égal à 1 ± 0,2. En effet au-delà d'un rapport de 1, le taux de conversion du glycérol à tendance à stagner et à ne pas augmenter sensiblement jusqu'à ce qu'un rapport d'environs 4. Ainsi de manière surprenante un rapport d'environ 1 est sensiblement aussi efficace qu'un rapport de 2 en termes de taux de conversion du glycérol ou en termes de sélectivité en acide glycolique.

Cette faible quantité de base induit une diminution de la quantité des sels produits lors d'une éventuelle étape ultérieure de neutralisation du milieu réactionnel qui peut être effectuée pour la récupération des produits de réaction et plus particulièrement de l'acide glycolique. Cette neutralisation peut se faire grâce à un ou plusieurs acides et notamment un acide inorganique tels que l'acide sulfurique. La quantité d'acide utilisé est généralement choisie pour neutraliser le milieu réactionnel, c'est-à-dire qu'elle est choisie de manière à obtenir une quantité d'ion hydronium équivalente à la quantité de base introduite à l'étape précédente.

Le glycérol utilisé dans le procédé selon l'invention peut être pur, en solution aqueuse et/ou en mélange avec d'autres composés. Le glycérol peut avantageusement être d'origine végétale (biosourcé), par exemple il peut provenir d'huile de colza ou de tournesol.

L'agent oxydant utilisé est préférablement l'oxygène, soit directement sous forme de dioxygène O₂, soit sous une autre forme telle que l'eau oxygénée H₂O₂. Le dioxygène peut être utilisé, pur ou en mélange (tel que de l'air), sous pression (par exemple de 1 à 10 bars, de préférence environ 5 bars) ou sous pression atmosphérique.

De préférence le milieu réactionnel est un milieu aqueux, comprenant plus de 50%, avantageusement plus de 80%, en poids d'eau.

La température réactionnelle peut être choisie dans une gamme de 50 à 150°C. De préférence elle est choisie dans une gamme de 55 à 110°C, avantageusement de 60 à 105°C, par exemple 100°C.

La réaction est avantageusement réalisée en agitation continue, par exemple en appliquant au mélange une rotation de 1500 à 2000 rpm.

Le catalyseur à base d'argent sur support est un catalyseur dont la proportion de la masse d'Ag par rapport à la masse du support peut varier de 10 à 0,2% (par exemple 5% ± 0,5), plus particulièrement de 5 à 0,5% Ag/Support et notamment de 1 à 2% Ag/Support. Un rapport d'environ 1,5 ± 0,1% est particulièrement avantageux. Ce catalyseur peut comprendre un autre métal mais un catalyseur ne comprenant essentiellement que de l'argent comme espèce métallique (c'est-à-dire non oxydée) est préféré pour mettre en oeuvre l'invention. Selon un mode de réalisation préféré de l'invention le catalyseur ne comprend comme espèce métallique que de l'argent et, éventuellement, des dopants, ces dopants étant décrits ci-après.

De préférence la quantité de catalyseur utilisée est choisie de manière à ce que le rapport massique glycérol/catalyseur dans le milieu réactionnel soit choisi dans une gamme allant de 2 à 100, de préférence de 5 à 20, plus particulièrement de 5 à 15.

Le support du catalyseur est choisi dans le groupe constitué par le CeO₂, l'Al₂O₃ basique éventuellement dopée avec un oxyde de calcium ou de cérium, une résine amphotère, le ZrO₂, et un mélange de ces matériaux. Par exemple le support peut-être de l'alumine ou une résine amphotère.

De manière particulièrement préférée le support comprend un oxyde choisi dans le groupe constitué par de l'alumine Al₂O₃ basique, de l'oxyde de cérium CeO₂, de l'oxyde de zirconium ZrO₂ et un mélange de ces oxydes. Le choix de tels supports permet d'obtenir un taux de conversion ainsi qu'une sélectivité élevé, en particulier supérieurs à 40%.

L'expression « alumine basique » désigne un matériau de formule Al₂O₃ présentant une acidité de Hammett comprise entre +7.8 et +8.9 (acidité mesurée respectivement par du rouge de crésol et du bleu de thymol). Cette même basicité peut être également mesurée par pH-métrie en ajoutant à une solution aqueuse 5g d'alumine basique dans 95g d'eau. Pour les alumines basiques utilisées le pH est supérieur à 9, tandis que dans les mêmes conditions une alumine présentera un pH compris entre 6 et 8 et qu'une alumine acide présentera un pH inférieur à 6. Une alumine basique permet d'obtenir des taux de conversion du glycérol bien supérieurs à ceux obtenus en utilisant de l'alumine gamma ou de l'alumine acide. Par exemple le taux de conversion peut-être multiplié par 3, 5, voir 6 et atteindre environs 40%. De même la sélectivité d'un catalyseur selon l'invention dont le support est une alumine basique peut être améliorée de 5, voire de 15% par rapport à la sélectivité d'une alumine non-basique.

Des résines non fortement acides sont utilisables comme support de catalyseur sont généralement des polymères synthétiques à base de molécules organiques. Ces résines sont communément à base de styrènes réticulés à l'aide de différentes quantités de divinylbenzène pour obtenir des polymères de tailles et de structures différentes. Des polymères à base d'acrylique sont également utilisables. Ces résines se présentent souvent sous forme de billes.

Des polymères, tels que les poly(styrène-divinylbenzène) et les polyacryliques, sont généralement fonctionnalisés par la présence de groupes acides (par exemple de l'acide sulfonique, ou carboxylique, ou leurs sels) ou basiques (par exemple des ions ammoniums quaternaires ou hydroxydes d'ammonium). Certaines résines présentent à la fois des groupes acides et basiques et sont appelées résines amphotères. Les résines utilisées comme support sont choisies dans le groupe constitué par les résines amphotères. Ce type de résines donne des résultats particulièrement satisfaisants.

Par résines non fortement acides on entend des résines présentant une acidité sur l'échelle de Hammett (Ho) ou (AH) supérieure à 2, de préférence supérieure ou égale à 6. Les résines basiques ou amphotères peuvent présenter de préférence un Ho supérieur ou égal à 6. Les résines amphotères de Ho égal à 7 ± 0,8 donnent de bons résultats.

Pour déterminer le Ho, la méthode de Hammett utilise une série d'indicateurs de couleurs. Environs 0,1-0,2 g d'un échantillon du support testé est immergée dans 5 ml d'un mélange de toluène anhydre et de cyclohexane (06:01 vol/vol). A cette suspension, 2-3 gouttes de solution à 0,1% d'un indicateur (voir ci-dessous) dans le toluène sont ajoutées. Cette suspension est mélangée et la couleur de la surface catalytique est observée. L'observation est poursuivie 20 min avec mélange occasionnel de l'échantillon.

Les indicateurs utilisés peuvent être choisis, par exemple, parmi les composés suivants:
- Cristal violet/violet de Paris/méthyl violet 10B : Ho = 0,8. Pour des valeurs inférieures, à savoir Hₒ < 0,8 la couleur est jaune, autour de 0,8 : vert et pour des valeurs supérieure Ho > 0,8 : bleue.
- Bleu de bromothymol : Ho = 7,2. La couleur acide est jaune, la forme de base, bleue.
- Rouge de phénol : Ho = 7,8. La forme acide est jaune, la forme basique est rouge.
- Le bleu de thymol bleu : Ho = 8,9. La forme classique acide est jaune, la forme basique est bleue.
- Carmin d'indigo : Ho = 12,2. La forme acide est bleue, la forme basique est jaune.
- Vert de Malachite: Ho = 13, 0. La forme classique acide est bleu-vert, la forme basique est incolore.

L'oxyde métallique utilisé en tant que support peut être choisi dans le groupe constitué par les oxydes de d'aluminium, de zinc, de zirconium, de lanthane, de magnésium, de calcium, de titane, de silice, et leurs mélanges. Un tel support peut notamment comprendre un oxyde modifié, comme de l'alumine basique, dopé par un ou plusieurs autres oxydes métalliques (par exemple Bi, Sn, Co, Ni, Zn, La, Mo, Mn, Ce, W, Ca....), induisant une évolution des propriétés acido-basiques de l'oxyde. Les oxydes de cérium, calcium, tungstène et molybdène sont des dopants préférés. Par «dopant» on entend que les quantités utilisées sont faibles, par exemple inférieures à 3 mmol de l'atome utilisé comme dopant et préférentiellement 1,5 mmol, par rapport à 1g de support. Le dopage du support est effectué de la manière suivante : le support est imprégné par une solution du sel de l'atome considéré (par exemple un sel d'ammonium ou de nitrate par exemple (Ce(NO₃)₃) ou Ca(NO₃)₂))) dans les proportions décrites précédemment, la poudre obtenue est ensuite séchée (par exemple par chauffage à 110°C pendant 24h) puis calcinée sous air (par exemple à 550°C pendant 3h). Avantageusement cette calcination est effectuée en suivant une montée en température d'environ 10°C/min. Les conditions exemplifiées ci-dessus peuvent évidemment varier. Une variation d'environ 25%, de préférence de 10%, peut être envisagée comme n'affectant pas les résultats de manière notable. Cependant il convient de choisir une température de calcination qui ne dégrade pas les catalyseurs.

En particulier, un dopage au cérium et/ou au calcium du support permet d'obtenir des taux de conversion supérieurs à ceux obtenus avec de l'argent pur. De plus un dopage au calcium permet d'atteindre une sélectivité en acide glycolique supérieure à celle obtenue avec de l'argent pur sur le même support.

Des oxydes particulièrement préférés sont l'alumine basique et des oxydes comprenant des groupes fonctionnelles amines (telles que APTES (3-aminopropyl)-triéthoxysilane (CAS# 919-30-2), APDEMS, APDMES et APTMS).

Selon un mode de réalisation préféré de l'invention le catalyseur est un catalyseur sur support de formule Ag/CeO₂, Ag/Al₂O₃ basique, éventuellement dopée par l'oxyde de calcium (CaO) ou par l'oxyde de cérium (CeO₂), ou Ag/IR45.

Le procédé selon l'invention permet d'obtenir une bonne sélectivité en acide glycolique. Cette sélectivité est généralement supérieure ou égale à 30%, elle peut être supérieure à 40%, et même supérieure à 50%.

Cette sélectivité est avantageusement combinée à un taux de conversion dépassant les 20%, par exemple de plus de 35% et avantageusement plus de 40%.

Un catalyseur à base d'argent sur support et notamment un catalyseur Ag/Al₂O₃ ou Ag/résine amphotérique est décrit dans la présente demande.

Un catalyseur tel que décrit est obtenu, ou susceptible d'être obtenu, par un procédé comprenant les étapes suivantes :
a) une mise en solution d'un composé à base d'argent; et
b) une mise en présence du composé à base d'argent avec le support, dans des conditions douces, pour fixer l'argent audit support.

De préférence, le composé à base d'argent est un oxyde d'argent, en particulier du nitrate d'argent.

De préférence le solvant du composé à base d'argent comprend un alcool tel que du méthanol.

De préférence le mélange est effectué en présence d'un solvant à base d'alcool tel que du méthanol. Ledit mélange est avantageusement chauffé.

De préférence l'étape b) entre le composé à base d'argent et le support comprend une étape de réduction de l'oxyde d'argent en argent métallique qui est alors disponible pour se fixer sur le support. Cette étape de réduction peut être effectuée par l'ajout d'un composé réducteur tel que du formaldéhyde ou encore de l'hydrazine (N₂H₄) ou du tétrahydruroborate de sodium (NaBH₄). Eventuellement cette étape est suivie d'une étape de chauffage.

De préférence l'étape de fixation comprend une étape de neutralisation de la solution. Cette étape peut être effectuée par l'ajout d'une base. Le pH peut alors être ajusté par cet ajout dans une gamme allant environs de 7 à 8.

De préférence le support est un des supports décrits précédemment et notamment il peut être choisi dans le groupe constitué par les résines et les supports à base d'oxyde comprenant des groupes fonctionnels aminés.

De préférence le support utilisé est déshydraté (séché) avant sa mise en suspension dans le solvant de la réaction.

Un tel catalyseur est susceptible d'être obtenu par le procédé de synthèse suivant :
Un support tel que décrit ci-dessus, soigneusement séché avant utilisation, est immergé dans du méthanol (V = 100 mL pour 10 g de support). A cette suspension, mélangée et chauffée (T ≈ 64°C), du nitrate d'argent dissout dans un mélange eau/méthanol est lentement ajouté au goutte à goutte afin d'obtenir une charge en argent d'environ 1,5 % en poids. Du formaldéhyde (formaldéhyde/métaux = 10 molaire) est alors ajouté afin de réduire l'argent. Le mélange ainsi obtenu est mélangé et chauffé (T ≈ 64°C) pendant 1 h. Le pH du mélange est ensuite ajusté à 7-8 par une solution d'hydroxyde de sodium. Le mélange est alors chauffé à la même température, sous agitation, pendant 20 minutes supplémentaires. Le mélange est finalement refroidit à température ambiante et filtré. Le catalyseur est ensuite lavé à l'eau distillée puis séché pendant 24 h à 110°C.

Un catalyseur selon l'invention est par exemple un catalyseur Ag/résine ayant une proportion d'argent métallique allant de 1 à 2% et en particulier d'environ 1,5%.

Un catalyseur ainsi que son procédé de fabrication et son utilisation en tant que catalyseur pour la synthèse de l'acide glycolique ou d'autres composés organiques est décrit dans la présente demande.

### Brève description des dessins

L'invention sera mieux comprise à la lecture des figures annexées, qui sont fournies à titre d'exemples et ne présentent aucun caractère limitatif, dans lesquelles :
La figure 1 représente la quantité de produits présents au cours de la réaction de conversion du glycérol en différents produits de synthèse décrite à l'exemple 3 en fonction du temps de réaction, cette réaction ayant lieu en présence d'un catalyseur 1,486wt% Ag/Al₂O₃ selon l'invention et tel que décrit à l'exemple 1.
La figure 2 : représente la quantité de produits présents au cours de la réaction de conversion du glycérol en différents produits de synthèse décrite à l'exemple 4 en fonction du temps de réaction, cette réaction ayant lieu en présence d'un catalyseur 1,486wt% Ag/IR45 selon l'invention et tel que décrit à l'exemple 2.
La figure 3 : représente la quantité de produits présents au cours de la réaction de conversion du glycérol en différents produits de synthèse de l'exemple 5.
La figure 4 : représente la quantité de produits présents au cours de la réaction de conversion du glycérol en différents produits de synthèse de l'exemple 6.

### Description détaillée de l'invention

### Exemple 1 : Synthèse d'un catalyseur sur alumine selon l'invention : 1.46 wt%Ag/Al₂O₃

9,9643g d'Al₂O₃ basique (Société Merck - BET ∼ 120 m2 / g) et 75 ml de MeOH ont été chauffés sous agitation à 67°C pendant 1 heure.

Passé ce délai, 0,2332g d'AgNO₃ (eq 0,1481g d'Ag) dissout dans 25 ml de MeOH a été ajouté lentement au mélange réactionnel. Le bêcher et l'entonnoir ont été lavés avec 30 ml d'eau distillée supplémentaires qui ont été également ajoutés au mélange réactionnel.

Le chauffage sous agitation a été poursuivi pendant 1h, puis 26,4ml (0,018 mole) de formaldéhyde (HCHO) ont été ajoutés. Jusqu'à cette étape la couleur de la suspension est blanchâtre.

Après 1h, 13.2ml d'une solution de NaOH (0,3 M) ont été introduits dans la solution, et celle-ci devient marron foncée. Le chauffage sous agitation est poursuivi pendant 1,5h.

Passé ce délai, la solution est lentement refroidie jusqu'à température ambiante et filtrée. Le filtrat obtenu est lavé avec 50 ml d'eau distillé puis séché à 110 °C pendant 12 h. La couleur de la poudre recueillie est grise foncée.

Le catalyseur obtenu est nommé selon la convention en vigueur selon son poids nominal de charge, c'est-à-dire le pourcentage en poids nominal d'argent utilisé rapporté au poids total du catalyseur.

### Exemple 2 : Synthèse d'un catalyseur sur résine selon l'invention : 1.486 Ag/IR45 (calculé pour la masse sèche de la résine)

Le support choisi est la IR45(OH), une résine formée de polymères polystyrène fortement connectés entre eux et fonctionnalisée par des groupements amine, commercialisée par la société Rhom & Haas -. Cette résine est une résine neutre ou amphotérique, Ho = 7,2 - 7,8. 9.1485g de résine IR45(OH) (6,7535g en masse sèche car 26,2% du poids sont perdus durant le séchage initial) est séchée puis ajoutée à 75 ml de MeOH, ce mélange est ensuite chauffé sous agitation à 67°C pendant 1 heure.

Passé ce délai, 0.1581g d'AgNO₃ (eq. 0,1004g d'Ag) dissout dans 25 ml de MeOH a été ajouté lentement au mélange réactionnel. Le bêcher et l'ampoule de coulée ont été lavés avec 30 ml d'eau distillée supplémentaires qui ont été également ajoutés au mélange réactionnel.

Le chauffage sous agitation a été poursuivi pendant 1 heure, puis 0,012 mole d'acide HCHO (9ml solution) a été ajoutée. Après 1 heure, 9ml de solution NaOH (0,3 M) ont été introduits dans la solution. Le chauffage sous agitation a été poursuivi pendant 1.5 heures.

Après cette période, la solution a été refroidie lentement jusqu'à température ambiante et filtrée. Le filtrat est lavé avec 50ml d'eau distillée puis séché à 110°C pendant 12 h. La couleur de la poudre recueillie est inhomogène est présente deux teintes : orange et verte.

### Exemple 3 (comparatif) : Utilisation du catalyseur de l'exemple 1 dans la synthèse de l'acide glycolique à partir du glycérol

L'oxydation du glycérol en phase liquide est effectué dans un réacteur de 300 mL en acier inoxydable équipé d'une turbine à gaz, de 4 contrepales, d'un thermocouple et d'un système d'alimentation en oxygène thermo-régulé. 200 mL d'une solution aqueuse de glycérol ([gly] = 0.3 M) sont chauffés à la température désirée de 60°C.

La soude (rapport molaire NaOH/Gly = 4) et le catalyseur (rapport massique gly/cat = 11 (g/g)) sont introduits dans le réacteur (t0) et le système est mis sous pression d'oxygène (5 bar) sous agitation continue (1500 rpm). La température et la pression partielle d'O₂ sont contrôlées en permanence tandis que l'échantillonnage s'effectue périodiquement. Les produits sont analysés par chromatographie en phase liquide à haute performances (HPLC) en utilisant un dispositif de type Agilent 1200 équipé d'une colonne Rezex ROA-Organic Acid H+ (300x7.8 mm) et d'un détecteur à indice de réfraction (RID). Une solution de H₂SO₄ (0,0025 M) dans de l'eau déminéralisée (0,5 mL·min-1) est utilisée comme éluant. L'identification et la quantification des produits obtenus sont réalisées par comparaison avec les courbes d'étalonnage correspondantes.

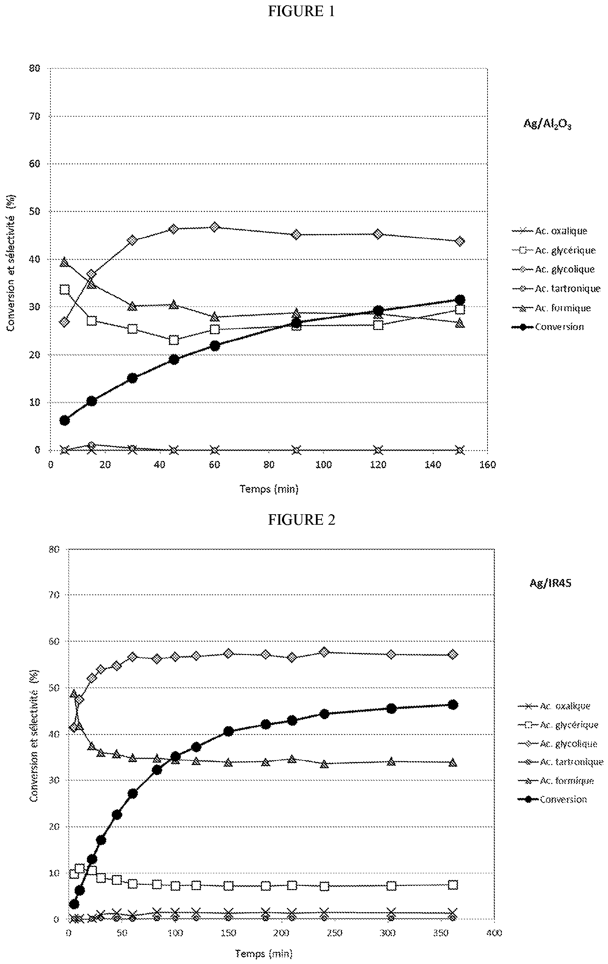

Le catalyseur composé d'environ 1% d'argent supporté sur alumine permet d'obtenir une sélectivité en acide glycolique (c'est-à-dire la quantité molaire d'acide glycolique formé par rapport au glycérol consommé), stable durant l'ensemble de l'expérience, proche de 50% (cf. FIGURE 1). Les deux autres produits formés, en quantité similaire, sont l'acide glycérique et l'acide formique (25% chacun). Le taux de conversion est supérieur à 30%.

### Exemple 4 : Utilisation du catalyseur de l'exemple 2 dans la synthèse de l'acide glycolique à partir du glycérol

L'oxydation du glycérol en phase liquide est effectué dans un réacteur de 300 mL en acier inoxydable équipé d'une turbine à gaz, de 4 contrepales, d'un thermocouple et d'un système d'alimentation en oxygène thermo-régulé. 200 mL d'une solution aqueuse de glycérol ([gly] = 0,3 M) sont chauffés à la température désirée à 100°C.

La soude (rapport molaire NaOH/Gly = 1) et le catalyseur (rapport massique gly/cat = 5,5 (g/g)) sont introduits dans le réacteur (t0) et le système est mis sous pression d'oxygène (5 bar) sous agitation continue (1500 rpm). La température et la pression partielle d'O2 sont contrôlées en permanence tandis que l'échantillonnage s'effectue périodiquement. Les produits sont analysés avec un dispositif HPLC Agilent 1200 équipé d'une colonne Rezex ROA-Organic Acid H+ (300x7, 8 mm) et d'un détecteur à indice de réfraction (RID). Une solution de H₂SO₄ (0,0025 M) dans de l'eau déminéralisée (0,5 mL·min-1) est utilisée comme éluant. L'identification et la quantification des produits obtenus sont réalisées par comparaison avec les courbes d'étalonnage correspondantes.

Dans cet exemple l'emploi d'une faible quantité de base (NaOH/Gly =1) permet d'obtenir de très bons résultats après 1 heure de réaction (35% de conversion et 55% de sélectivité en acide glycolique, cf. FIGURE 2). L'emploi de support amphotère et/ou neutre est donc préféré.

L'utilisation de résine purement acide (IRA120 - Fluka - AH = 0.8) conduit à la transformation du glycérol en acide glycérique. L'utilisation d'une résine fortement basique (IRA400 Fluka -AH > 14) transforme le glycérol en acide glycérique et en acide glycolique dans les mêmes proportions (Sélectivité = 30%).

### Exemple 5 : Utilisation du catalyseur de l'exemple 1 dans la synthèse de l'acide glycolique à partir du glycérol en utilisant une concentration de base réduite

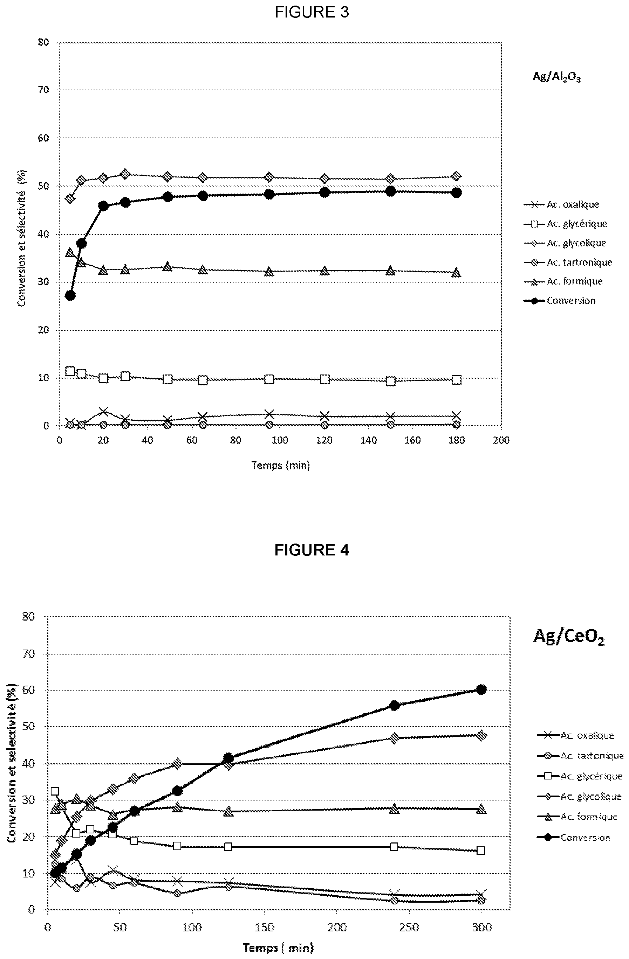

L'exemple 4 a été répété avec le catalyseur de l'exemple 1 (1,486wt%Ag/Al2O3) et les résultats obtenus, présentés dans la FIGURE 3, sont proches de ceux de l'exemple 4.

### Exemple 6 : Méthode de synthèse d'acide glycolique en utilisant un catalyseur argent sur support CeO2.

### Préparation du support CeO2 :

On a réalisé une solution aqueuse de nitrate de cérium (0,5 M) à partir de cerium (III) nitrate hexahydrate (puriss. p.a., ≥99.0% Fluka). La solution obtenue a été additionnée goutte à goutte dans une solution (en excès) de triéthylamine (1,5 M) diluée dans le méthanol. Le précipité d'hydroxyde obtenu a été récupéré par filtration. Il a été ensuite lavé et rincé par de l'eau et du méthanol plusieurs fois. Il a été séché à l'étuve à 100°C. Le solide a ensuite été broyé sous la forme d'une fine poudre puis calciné sous air à 500°C 4h.

La synthèse du catalyseur a été effectuée selon le mode opératoire de l'exemple 1. La synthèse de l'acide glycolique a été effectué selon le mode opératoire de l'exemple 3, c'est à dire à 60°C, 5 bars d'O2, 0.5 g de catalyseur, 1500 rpm, 200 ml d'une solution de glycérol pur (0.3 M) et un rapport molaire NaOH/glycerol = 4.

Ce catalyseur permet d'obtenir une sélectivité en acide glycolique (c'est-à-dire la quantité molaire d'acide glycolique formé par rapport au glycérol consommé), stable durant l'ensemble de l'expérience, proche de 50% (cf. FIGURE 4). Le taux de conversion est supérieur et proche de 60%.

L'invention n'est pas limitée aux modes de réalisation présentés et d'autres modes de réalisation apparaîtront clairement à l'homme du métier. L'invention est définie par les revendications.

## Revendications

1. Procédé de synthèse d'acide glycolique, ou d'un de ses sels, comprenant l'étape suivante :
- une mise en contact dans un milieu réactionnel de glycérol et d'un agent oxydant en présence d'un catalyseur à base d'argent sur support, ledit support comprenant un matériau choisi dans le groupe constitué par le CeO₂, l'Al₂O₃ basique éventuellement dopée avec un oxyde de calcium ou de cérium, une résine amphotère, le ZrO₂, et un mélange de ces matériaux ; et
dans lequel une base produisant des ions hydroxyles est présente dans le milieu réactionnel, et le rapport molaire d'ions hydroxyles et de glycérol [OH]/[gly] mis en présence étant de 0,5 à 1,2 ;
et ledit catalyseur ne comprenant essentiellement que de l'argent comme espèce métallique.

2. Procédé selon la revendication 1 dans lequel ledit catalyseur est un catalyseur sur support de formule Ag/CeO₂, Ag/Al₂O₃ basique, éventuellement dopée à l'oxyde de calcium ou à l'oxyde de cérium, ou Ag/IR45 ; IR45 étant une résine formée de polymères polystyrène fortement connectés entre eux et fonctionnalisée par des groupements amine ladite résine présentant une valeur Ho de 7,2 à 7,8

3. Procédé selon la revendication 1 ou 2, dans lequel le catalyseur ne comprend comme espèce métallique que de l'argent.

4. Procédé selon l'une quelconque des revendications précédentes, dans lequel le pH du milieu réactionnel est supérieur ou égal à 8.

5. Procédé selon l'une quelconque des revendications précédentes, dans lequel ledit agent oxydant est l'oxygène.

6. Procédé selon l'une quelconque des revendications précédentes, dans lequel la température du milieu réactionnel est choisie dans une gamme allant de 50 à 150°C.

7. Procédé selon l'une quelconque des revendications précédentes, dans lequel le catalyseur à base d'argent sur support est un catalyseur dont la proportion en poids d'argent par rapport au support varie de 10 à 0,2%.

8. Procédé selon la revendication 7, dans lequel le catalyseur est constitué d'argent et dudit support et dans lequel le rapport massique glycérol/catalyseur dans le milieu réactionnel est choisi dans une gamme allant de 2 à 100.

9. Procédé selon l'une quelconque des revendications précédentes, dans lequel le glycérol est d'origine végétale.

10. Procédé selon l'une quelconque des revendications précédentes, dans lequel les quantités d'oxydes de calcium ou de cérium éventuellement utilisées sont inférieures à 3 mmol de l'atome utilisé par rapport à 1g de support.

11. Procédé selon l'une quelconque des revendications 1 à 10, dans lequel ladite mise en contact est réalisée en agitation continue du milieu réactionnel.

12. Procédé selon la revendication 5, dans lequel l'oxygène est du dioxygène O₂ ou de l'eau oxygénée H₂O₂.

## Patentansprüche

1. Verfahren zur Synthese von Glycolsäure oder eines Salzes davon, umfassend den folgenden Schritt
- Kontaktieren von Glycerin und einem Oxidationsmittel in einem Reaktionsmedium in Gegenwart eines auf Silber basierenden Katalysators auf einem Träger, wobei der Träger ein Material umfasst, ausgewählt aus der Gruppe, bestehend aus CeO₂, basischem Al₂O₃, das gegebenenfalls mit einem Calcium- oder Ceroxid dotiert ist, einem amphoteren Harz, ZrO₂ und einem Gemisch dieser Materialien, und
wobei eine Hydroxylionen-produzierende Base im Reaktionsmedium vorhanden ist und das Molverhältnis von Hydroxylionen und zusammengeführtem Glycerin [OH]/[Gly] 0,5 bis 1,2 beträgt,
und wobei der Katalysator im Wesentlichen nur Silber als Metallspezies umfasst.

2. Verfahren nach Anspruch 1, wobei der Katalysator ein Trägerkatalysator der Formel Ag/CeO₂, Ag/basisches Al₂O₃, gegebenenfalls dotiert mit Calciumoxid oder Ceroxid, oder Ag/IR45 ist, wobei IR45 ein Harz ist, das aus stark miteinander verbundenen Polystyrolpolymeren gebildet und durch Aminogruppen funktionalisiert ist, wobei das Harz einen Ho-Wert von 7,2 bis 7,8 aufweist.

3. Verfahren nach Anspruch 1 oder 2, wobei der Katalysator als Metallspezies nur Silber umfasst.

4. Verfahren nach einem der vorangehenden Ansprüche, wobei der pH-Wert des Reaktionsmediums größer oder gleich 8 ist.

5. Verfahren nach einem der vorangehenden Ansprüche, wobei das Oxidationsmittel Sauerstoff ist.

6. Verfahren nach einem der vorangehenden Ansprüche, wobei die Temperatur des Reaktionsmediums aus einem Bereich von 50 bis 150°C ausgewählt ist.

7. Verfahren nach einem der vorangehenden Ansprüche, wobei der auf Silber basierende Trägerkatalysator ein Katalysator ist, dessen Gewichtsanteil an Silber zum Träger von 10 bis 0,2% variiert.

8. Verfahren nach Anspruch 7, wobei der Katalysator aus Silber und dem Träger besteht und wobei das Gewichtsverhältnis von Glycerin/Katalysator im Reaktionsmedium aus einem Bereich von 2 bis 100 ausgewählt ist.

9. Verfahren nach einem der vorangehenden Ansprüche, wobei das Glycerin pflanzlichen Ursprungs ist.

10. Verfahren nach einem der vorangehenden Ansprüche, wobei die Mengen an Calcium- oder Ceroxiden, falls vorhanden, weniger als 3 mMol des verwendeten Atoms in Bezug auf 1 g Träger betragen.

11. Verfahren nach einem der Ansprüche 1 bis 10, wobei das Kontaktieren durch kontinuierliches Bewegen des Reaktionsmediums durchgeführt wird.

12. Verfahren nach Anspruch 5, wobei der Sauerstoff molekularer Sauerstoff O₂ oder Wasserstoffperoxid H₂O₂ ist.

## Claims

1. Process for preparing glycolic acid, or one of its salts, comprising the following step:
- bringing glycerol and an oxidizing agent into contact in a reaction medium in the presence of a silver-based catalyst on support, said support comprising a material selected from the group consisting of CeO₂, basic Al₂O₃ optionally doped with a calcium or cerium oxide, an amphoteric resin, ZrO₂, and a mixture of these materials, and
wherein a base producing hydroxyl ions is present in the reaction medium, and the molar ratio of hydroxyl ions and glycerol [OH]/[gly] brought in contact being 0.5 to 1.2, and said catalyst mainly comprising only silver as metal species.

2. Process according to claim 1, wherein said catalyst is a catalyst on support of formula Ag/CeO₂, basic Ag/Al₂O₃, optionally doped with a calcium or cerium oxide, or Ag/IR45, IR45 being a resin formed of polystyrene polymers highly connected to one another and functionalised by amine groups, said resin having an Ho value of 7.2 to 7.8

3. Process according to claim 1 or 2, wherein the only metal species contained in the catalyst is silver.

4. Process according to any one of the preceding claims, wherein the pH of the reaction medium is greater than or equal to 8.

5. Process according to any one of the preceding claims, wherein said oxidising agent is oxygen.

6. Process according to any one of the preceding claims, wherein the temperature of the reaction medium is chosen in a range from 50 °C to 150 °C.

7. Process according to any one of the preceding claims, wherein the silver-based catalyst on support is a catalyst whose proportion by weight of silver relative to the support varies from 10 % to 0.2 %.

8. Process according to claim 7, wherein the catalyst consists of silver and said support and wherein the glycerol/catalyst mass ratio in the reaction medium is chosen in a range from 2 to 100.

9. Process according to any one of the preceding claims, wherein the glycerol is of vegetable origin.

10. Process according to any one of the preceding claims, wherein the quantities of calcium or cerium oxides optionally used are less than 3 mmol of the atom used per 1 g of support.

11. Process according to any one of claims 1 to 10, wherein said bringing into contact is carried out by continuous stirring of the reaction medium.

12. Process according to claim 5, wherein the oxygen is dioxygen O₂ or hydrogen peroxide H₂O₂.
